# EUROPÄISCHE PATENTSCHRIFT

(11) **EP 0 716 701 B1**
(45) Veröffentlichungstag und Bekanntmachung des Hinweises auf die Patenterteilung: **07.12.2005**
(21) Anmeldenummer: 94926907.0
(22) Anmeldetag: 30.08.1994
(51) Int. Cl.: C12N 15/54, C12N 15/63, C12N 9/12, C12N 5/10, C07K 16/40, A61K 38/45, A61K 39/395, A61K 48/00

(54) **SERIN-THREONIN-KINASE ENTHALTENDES MITTEL ZUR TUMORTHERAPIE UND TUMORDIAGNOSE**
AGENT COMPRISING SERINE-THREONINE KINASE FOR THE DIAGNOSTIC AND THERAPY OF TUMORS
AGENT CONTENANT DE LA SERINE-THREONINE KINASE POUR LE DIAGNOSTIC ET LA THERAPIE DES TUMEURS

(30) Priorität: 30.08.1993 DE 4329177
(43) Veröffentlichungstag der Anmeldung: 19.06.1996
(73) Patentinhaber: Rübsamen-Waigman, Helga, Prof.Dr., 65812 Bad Soden (DE); Strebhardt, Klaus, Prof. Dr., 60489 Frankfurt (DE)
(72) Erfinder: STREBHARDT, Klaus, D-60489 Frankfurt (DE); RÜBSAMEN-WAIGMANN, Helga, D-65812 Bad Soden (DE); HOLTRICH, Uwe, D-65779 Kelkheim (DE)
(74) Vertreter: Böhm, Brigitte
(86) Internationale Anmeldenummer: PCT/EP1994/002863
(87) Internationale Veröffentlichungsnummer: WO 1995/006734

(56) Entgegenhaltungen:
- PROCEEDINGS OF THE NATIONAL ACADEMY OF SCIENCES OF USA., Bd.90, Nr.11, 1. Juni 1993, WASHINGTON US Seiten 4882 - 4886 FIONA J. CLAY ET AL. 'Identification and cloning of a protein kinase-encoding mouse gene, Plk, related to the polo gene of Drosophila'
- EMBL Datenbank eingang HSPLK1 Zugriffsnummer X73458; 1 Juli 1993 GOLSTEYN R.M. ET AL.: 'Cloning and characterization of a novel human protein
- GENES & DEVELOPMENT, Bd.5, Nr.12A, Dezember 1991 Seiten 2153 - 2165 S. LLAMAZARES ET AL. 'Polo encodes a protein kinase homolog required for mitosis in Drosophila'
- CELL GROWTH & DIFFERENTIATION, Bd.5, Nr.3, März 1994 Seiten 249 - 257 RYOGI HAMANAKA ET AL. 'Cloning and characterization of human homologues of the Drosophila polo serine-threonine kinase'
- PROCEEDINGS OF THE NATIONAL ACADEMY OF SCIENCES OF USA., Bd.91, Nr.5, 1. März 1994, WASHINGTON US Seiten 1736 - 1740 UWE HOLTRICH ET AL. 'Induction and down-regulation of PLK, a human serine/threonine kinase expressed in proliferating cells and tumors'
- EMBL Datenbank eingang HSPLKSTK Zugriffsnummer X75932; 22 März 1994 HOLTRICH U. ET AL.: 'Induction and down- regulation of PLK, a human

## Beschreibung

Die vorliegende Erfindung betrifft die Verwendung einer Serin-Threonin-Kinase, als aktiven Bestandteil eines Arzneimittels zur Verwendung bei der Tumortherapie oder als diagnostisches Mittel zur Verwendung in der Tumordiagnostik.

Die Aktivierung intrazellulärer biochemischer Netzwerke als Antwort auf externe Stimuli führt zur koordinierten Kontrolle des Wachstums und der Differenzierung in Eukaryonten. Protein-Kinasen sind als Bestandteile von vielen Signaltransduktionswegen bekannt. Hierbei phosphorylieren diese Kinasen ihre normalen physiologischen Substrate und werden in ihrer enzymatischen Aktivität durch die Interaktion mit anderen Kinasen und Phosphatasen reguliert. Die Identifikation einer großen Zahl von Protein-Kinasen bei vielen eukaryontischen Zellen von Säugetieren, Hefe und Drosophila macht es wahrscheinlich, daß grundlegende zelluläre Differenzierungs- und Wachstumsprozesse in einem breiten Spektrum von Organismen durch gleiche Mechanismen gesteuert werden.

In Eukaryonten phosphorylieren alle bisher bekannten Protein-Kinasen die Hydroxyaminosäuren Serin, Threonin oder Tyrosin. Die Phosphorylierung durch diese Kinasen spielt für die Kontrolle der Mitose und der zellulären Differenzierung eine hervorgehobene Rolle. Rezeptoren für zahlreiche Polypeptidwachstumsfaktoren sind transmembranöse Tyrosinkinasen, die ihrerseits Serin/Threonin-Kinasen wie Protein Kinase C, MAP-Kinase und p74raf phosphorylieren. Die zentrale Komponente der Zellzyklusmaschinerie ist die Serin/Threonin-Kinase p34cdc2, die ursprünglich als Produkt der Mitosegene cdc2 (cell division cycle) aus Schizosaccharomyces pombe und CDC28 aus Saccharomyces cerevisiae isoliert worden ist. Die Aktivität von p34cdc2 wird durch die Wechselwirkung mit Cyclinen reguliert. In der G1-Phase, dem Beginn des Zellzyklus, ist p34cdc2 nicht mit Cyclinen assoziiert und hat keine Kinaseaktivität. Werden Zellen mit ausreichenden Nährstoffen versorgt, akkumuliert Gl-Cyclin, das durch Assoziation die Kinaseaktivität von p34cdc2 initiiert. Hierdurch wird "Start" überschritten und der Übergang in den Zellzyklus (DNA-Replikation, Bildung des MTOC, microtubule organizing centre) eingeleitet. Hierbei beginnt auch die Synthese von Cyclin B, das dann an p34cdc2 bindet. Dieser Komplex ist inaktiv, denn die Bindung von Cyclin B induziert die Phosphorylierung von p34cdc2 an Tyrosin 15, wodurch die Kinaseaktivität inhibiert wird. Der inaktive Komplex, der auch als preMPF (maturation promoting factor) bezeichnet wird, ist auch an Threonin 160 phosphoryliert. Diese Phosphorylierung ist für die MPF-Aktivität notwendig, aber nicht ausreichend, um den inhibitorischen Effekt der Tyrosinphosphorylierung aufzuheben. Die anschließende Dephosphorylierung von p34cdc2 während der späten G2-Phase des Zellzyklus aktiviert MPF und führt zur Induktion der Mitose (M-Phase). Die posttranslationalen Reaktionen, die einer komplexen physiologischen Steuerung unterliegen, spielen eine wesentliche Rolle für die zeitliche Regulation der MPF-Aktivität. Die Protein-Tyrosin-Kinase weel wurde ursprünglich aus Schizosaccharomyces pombe isoliert und inhibiert über den beschriebenen Mechanismus den Eintritt in die Mitose, während das Produkt des cdc25-Gens den Beginn der Mitose fördert. Aktives MPF aktiviert die Tyrosin-Phosphatase und inhibiert die Protein-Tyrosin-Kinase, die p34cdc2 modifizieren, wodurch MPF explosionsartig vollständig aktiviert wird, so daß Zellen sehr schnell und irreversibel in die Mitose getrieben werden.

Neueste Untersuchungen des Zellzyklus zeigen, daß wesentliche Regulatoren des Zellzyklus an der Krebsentstehung beteiligt sind. Dies ist deshalb nicht überraschend, weil fortwährende Proliferation von Zellen ein herausragendes Merkmal von Tumoren ist. Änderungen am Cyclin A, das sowohl an p34cdc2 als auch der p34cdc2-verwandten Protein-Kinase bindet, können die Transformation von Zellen hervorrufen. Das Cyclin A-Gen ist Integrationsort für ein Fragment des Hepatitis B-Virusgenoms in einem humanen Hepatokarzinom. Darüber hinaus ist Cyclin A mit dem transformierenden Protein E1A in Adenovirus-transformierten Zellen assoziiert. Möglicherweise ist Cyclin A ein Zielprotein von E1A, denn es ist in der S-Phase mit dem Transkriptionsfaktor E2F in einem Komplex assoziiert, der geringere Transkriptionsaktivität hat als freies E2F. Ein weiterer Bestandteil dieses Komplexes ist p34cdc2. Auf diese Weise wird der Bezug zur Genexpression hergestellt. E1A kann diesen Komplex zerstören, wodurch E2F freigesetzt wird. Somit können bestimmte Gene reguliert werden, die für den transformierten Phänotyp bedeutsam sind.

Darüber hinaus gibt es weitere Beziehungen zwischen Onkoproteinen, Tumorsuppressorgenprodukten und Cyclin-p34cdc2-Komplexen. Das mos-Onkoprotein ist ebenfalls eine Serin/Threonin-Kinase. Das c-mos-Protein aus Xenopus ist eine Komponente des zytostatischen Faktors, der für die Stabilisierung des aktivierten Cyclin B-p34cdc2 in wachstumsgehemmten Xenopus-Eiern erforderlich ist. Die Rolle des mos-Proteins ist jedoch noch unklar, da es den Komplex in vivo nicht zu phosphorylieren scheint. Einerseits stabilisiert c-mos den aktivierten Cyclin B-p34cdc2-Komplex, wodurch die Zellen in ihrer Mitoseaktivität gehemmt werden, und andererseits fördert es den Zellzyklus. Bisherige Untersuchungen belegen die Annahme, daß das unterschiedliche Verhalten der Zellzyklusmaschinerie durch die Menge an mos-Protein gesteuert wird.

Verschiedene Onkoproteine wie z.B. die src- und abl-Protein-Tyrosin-Kinasen werden im Rahmen der Mitose ebenfalls von der Serin/Threonin-Kinase p34cdc2 phosphoryliert. In der src-Familie wird die mitotische Phosphorylierung von erhöhter Kinaseaktivität begleitet. Die Produkte der Tumorsuppressorgene RB und p53 bilden ebenfalls Komplexe mit Cyclin-p34cdc2 und werden hierbei phosphoryliert. Im Falle von RB scheint die Phosphorylierung notwendig zu sein, um die RB-Funktion zu inaktivieren, so daß die Zellen von G1 in die S-Phase fortschreiten können. Als Konsequenz der aberranten Expression des Cyclin-p34cdc2-Komplexes ergibt sich, daß solche Tumorsuppressorgenprodukte in ihrem phosphorylierten inaktiven Zustand konserviert werden, was die ungezügelte Zellteilung zur Folge hat.

Die Verbindung zwischen der Funktion von Serin/Threonin-Kinasen des Zellzyklus und der Transformation zeigt deutlich, daß geringfügige Störungen der Mitoseabläufe eine kritische Rolle bei der Krebsentstehung spielen. Da sich die Karzinogenese als Mehrstufenprozeß darstellt, kooperieren Mutationen in Zellzyklus-Regulatoren mit Mutationen, die Proto-Onkogene aktivieren oder Tumorsuppressorgene inaktivieren.

Aufgrund der enormen klinischen Bedeutung von Serin-Threonin-Kinasen bestand somit ein Bedürfnis, neue Arzneimittel und diagnostische Mittel bereitzustellen.

Ein Gegenstand der Erfindung ist daher ein Arzneimittel zur Verwendung bei der Tumortherapie, dadurch gekennzeichnet, dass es als aktiven Bestandteil ein PLK-Protein enthält, welches
(a) die in SEQ ID NO: 2 dargestellte Aminosäuresequenz oder
(b) Teile oder/und Varianten der Sequenz aus (a), welche auf Aminosäureebene eine Homologie von mindestens 95% gegenüber der Sequenz aus (a) aufweisen und hinsichtlich ihrer biologischen oder/und immunologischen Aktivität dem in SEQ ID NO. 1 dargestellten Protein entsprechen, umfasst,
oder einen gegen ein solches PLK-Protein gerichteten Antikörper oder eine Nukleinsäure, welche für ein solches PLK-Protein codiert, gegebenenfalls zusammen mit pharmazeutisch üblichen Hilfs-, Träger-, Füll- oder Verdünnungsmitteln.

Ein weiterer Gegenstand der Erfindung ist ein diagnostisches Mittel zur Verwendung in der Tumordiagnostik oder bei der Bestimmung der Aktivität von Lymphozyten, dadurch gekennzeichnet, dass es als aktiven Bestandteil ein PLK-Protein, welches (a) die in SEQ ID NO. 2 dargestellte Aminosäuresequenz oder (b) Teile oder/und Varianten der Sequenz aus (a), welche auf Aminosäureebene eine Homologie von mindestens 95% gegenüber der Sequenz aus (a) aufweisen und hinsichtlich ihrer biologischen oder/und immunologischen Aktivität dem in SEQ ID NO. 1 dargestellten Protein entsprechen, umfasst, einen Antikörper gegen ein solches PLK-Protein oder eine Nukleinsäure, welche für ein solches PLK-Protein codiert, enthält.

Ebenfalls ein Gegenstand der vorliegenden Erfindung ist die Verwendung eines solchen PLK-Proteins oder des dafür codierenden Gens oder eines gegen das Protein gerichteten Antikörpers im Rahmen der Tumordiagnostik. So haben Versuche gezeigt, daß die Expression des PLK-Gens in Tumoren und korrespondierenden tumorfreien Referenzgeweben unterschiedlich ist. Es besteht daher die Möglichkeit, aus der Expressionshöhe des Gens bzw. des Proteins auf die Teilungsaktivität von Zellen zu schließen.

Außerdem betrifft die Erfindung ein diagnostisches oder therapeutisches Mittel zur Verwendung bei der Tumordiagnostik bzw. Tumortherapie auf Basis eines Antikörpers, der gegen den aktiven Bestandteil eines PLK-Proteins, welches:
(a) die in SEQ ID NO: 2 dargestellte Aminosäuresequenz oder
(b) Teile oder/und Varianten der Sequenz aus (a), welche auf Aminosäureebene eine Homologie von mindestens 95% gegenüber der Sequenz aus (a) aufweisen und hinsichtlich ihrer biologischen oder/und immunologischen Aktivität dem in SEQ ID NO. 1 dargestellten Protein entsprechen, umfasst, gerichtet ist oder einer Nukleinsäure, welche für ein solches Protein codiert (z.B. als Antisense-Nukleinsäure zur Unterdrückung der Genexpression). Ein derartiges Mittel kann gegebenenfalls bekannte pharmazeutische Verdünnungs-, Füll-, Träger- und Hilfsmittel enthalten.

Ferner betrifft die Erfindung die Verwendung eines diagnostischen Mittels zur Bestimmung der Aktivität von Lymphozyten. Dieses Arzneimittel kann z.B. extrakorporal an entnommenen Blutproben angewendet werden. Besonders geeignet ist dieses diagnostische Mittel zum Nachweis von Störungen des Immunsystems, insbesondere bei Autoimmunerkrankungen oder Immunmangelsyndromen einschließlich AIDS.

Vorzugsweise ist das PLK-Protein ein aus dem Menschen erhältliches Protein, d.h. es ist das in SEQ ID NO.1 und NO.2 gezeigte Protein oder eine natürlich vorkommende humane Variante davon.

Das Protein umfasst Teile der in SEQ ID NO:1 und 2 dargestellten Aminosäuresequenz. Die Erfindung betrifft vorzugsweise ein PLK-Protein, welches die in SEQ ID NO:1 und 2 dargestellte Aminosäuresequenz enthält, es kann jedoch auch Varianten dieser Sequenz enthalten. Unter dem Begriff "Variante" im Sinne der vorliegenden Erfindung sind Sequenzen zu verstehen, die durch Substitution, Deletion oder/und Insertion einzelner Aminosäuren oder kurzer Aminosäureabschnitte sich von der in SEQ ID NO:1 und 2 dargestellten Aminosäuresequenz unterscheiden. Unter den Begriff "Variante" fallen sowohl natürlich vorkommende allelische Variationen des PLK-Proteins, sowie durch rekombinante DNA-Technologie (insbesondere durch in vitro-Mutagenese mit Hilfe von chemisch synthetisierten Oligonukleotiden) erzeugte Proteine, die hinsichtlich ihrer biologischen oder/und immunologischen Aktivität dem in SEQ ID NO:1 gezeigten Protein entsprechen.

Erfindungsgemäß im Arzneimittel bzw. diagnostischen Mittel enthaltene Proteine zeichnen sich dadurch aus, daß auf Aminosäureebene eine Homologie von mindestens 95 %, gegenüber der in SEQ ID NO:1 und 2 dargestellten Aminosäuresequenz aufweisen.

Das hier beschriebene, für das im erfindungsgemäßen Arzneimittel bzw. diagnostischen Mittel enthaltene PLK-Protein kodierende Gen PLK (polo-like-kinase) wurde aus einer cDNA-Bank, basierend auf humaner Lungentumor-RNA (Plattenepithelkarzinom), isoliert.

### Expression des PLK-Gens in proliferierenden Zellen und Geweben

Für eine Northern-Blot-Untersuchung wurde RNA aus folgenden humanen adulten Geweben isoliert: Lunge, Leber, Herz, Gehirn, Pankreas, Niere, Plazenta, Skelettmuskel, Ösophagus, Kolon, Magen und Milz. Nur in Plazenta und Kolon, also in Geweben, die einen größeren Prozentsatz von proliferierenden Zellen enthalten, wurde PLK-Expression nachgewiesen. Die Länge der PLK-mRNA beträgt ca. 2,3 kb. Im folgenden wurde der Hypothese nachgegangen, daß PLK-Expression mit der Proliferation von Zellen korreliert: Hierzu wurden humane Tumoren inklusive umgebender normaler Referenzgewebe untersucht. Starke Expression wurde in Tumoren folgender Organe gefunden: Lunge, Mamma, Ösophagus, Magen und Darm sowie in Leiomyosarkomen und Non-Hodgkin-Lymphomen. In einem Stichprobenkollektiv von 48 Lungentumoren war PLK in 84 % der Proben stark exprimiert. In den restlichen 16 % der Tumoren war PLK gering oder gar nicht exprimiert. Southern-Blot-Untersuchungen von restringierter DNA aus Tumoren und gesunden Referenzgeweben zeigten mit einer PLK-spezifischen Sonde keine Unterschiede.

Aufschluß über den Tumortyp und den PLK-Expressionsstatus gibt Tabelle 1:

**Tabelle 1:**

| Lunge | positiv | negativ |
|---|---|---|
| Plattenepithelkarzinom | 24 | 2 |
| Adenokarzinom | 10 | 2 |
| großzellige Karzinome | 6 | 1 |
| kleinzellige Karzinome | 3 | 0 |
| Referenzen (adulte humane Lunge) | 0 | 48 |
| Lebermetastase | 1 | 0 |
| Leiomyosarkom | 1 | 0 |
| Non-Hodgkin-Lymphom | 1 | 0 |

| Mamma | | |
|---|---|---|
| Mammakarzinome | 7 | 1 |
| Referenzen (normales Mammagewebe) | 0 | 8 |

| Darm | | |
|---|---|---|
| Kolorektale Karzinome | 3 | 0 |
| Referenzen (normales Kolongewebe) | 3 | 0 |

Neben humanen Geweben und primären Zellen wurden folgende Zellinien auf PLK-Expression getestet:
HELA-Zellinie (Zelltyp: Zervix-Karzinom),
Lungenepithel-Zellinie (Zelltyp: Lungen-Karzinom),
A431-Zellinie (Zelltyp: Epidermoides Karzinom),
HEP-38-Zellinie (Zelltyp: Hepatozelluläres Karzinom),
A498-Zellinie (Zelltyp: Nierenkarzinom),
BT 20-Zellinie (Zelltyp: Mammakarzinom),
T47D-Zellinie (Zelltyp: ductales Mammakarzinom),
SKBR3-Zellinie (Zelltyp: Adenokarzinom der Mamma),
NCF7-Zellinie (Zelltyp: Adenokarzinom der Mamma) und
HUV-EC-C-Zellinie (Zelltyp: Nabelschnurvenen-Endothelzellen).

PLK-Transkripte waren in sämtlichen untersuchten Zellinien nachweisbar.

Es wurde gefunden, daß die Expression des PLK-Gens mit der Proliferationsaktivität korreliert. Damit ergibt sich die Möglichkeit, die Menge von PLK-Genprodukten (mRNA bzw. Protein) als Maß für die Teilungsaktivität einer Zelle und damit als Proliferationsmarker für die Analyse von humanen Neoplasien einzusetzen. Wie aus Tabelle 1 hervorgeht, ist eine erhöhte PLK-Expression in einem sehr hohen Prozentsatz der untersuchten Tumoren nachweisbar. Somit handelt es sich bei PLK um einen universell einsetzbaren Proliferationsmarker.

### Mitogen-induzierte Expression von PLK in Lymphozyten

Ruhende Lymphoyzten aus peripherem Blut exprimieren das PLK-Gen nicht. Durch Zugabe von PHA (Phytohaemagglutinin) oder PHA/IL-2 (Interleukin-2) werden Lymphozyten stimuliert und es kommt zur Induktion der Expression von PLK. Demzufolge kann die Expression von PLK auch als Maß der Aktivierung von Lymphozyten genommen werden. In einem weiteren Experiment wurde die Zellinie A431 zunächst in serumhaltigem und dann in serumfreiem Medium kultiviert. Die Expression von PLK ging im Laufe der 5 Tage ohne Serum zurück. Nach Zugabe von Serum stieg die PLK-Expression wieder stark an.

### Abnahme der PLK-Expression in humanen Makrophagen

Makrophagen aus peripherem Blut sind Zellen, wenn überhaupt, mit sehr geringer Proliferationsaktivität. Je nach Spender hatten Makrophagen nach 15 Tagen in Kultur keine oder nur sehr geringe PLK-Expression. Nach Zugabe von LPS (Lipopolysaccharid) wurde die PLK-Expression innerhalb von 24 Stunden komplett abgeschaltet.

### Korrelation der PLK-Expression mit der Prognose von Tumorpatienten

Weiterhin wurde gefunden, daß die Prognose, insbesondere die Lebenserwartung von Tumorpatienten mit der Expression von PLK korreliert. Hierzu wurde ein größeres Kollektiv von Lungentumorpatienten untersucht und gefunden, daß mit steigender PLK-Expression die Lebenserwartung der Patienten sinkt. Damit kann PLK als diagnostischer Tumormarker auch für die Prognose eingesetzt werden. Die Besonderheit von PLK ist darin zu sehen, daß eine erhöhte Expression in mehr als 80 % der untersuchten Patienten auftritt, so daß PLK ein sehr guter Tumormarker ist, der mit hoher Wahrscheinlichkeit das Vorhandensein von Tumoren anzeigt.

Die Fähigkeit von PLK als Prognosemarker eingesetzt zu werden, gilt nicht nur für Lungentumoren, sondern auch für andere Tumoren wie etwa Brustkrebs etc.

Ein Aspekt der Erfindung ist somit ein Verfahren zur Bestimmung der Teilungsaktivität von Zellen, insbesondere von humanen Zellen, wobei man die Expressionsstärke des für das PLK-Protein codierenden Gens in der betreffenden Zelle ermittelt, z.B. auf Transkriptionsebene durch Northern-Blot-Verfahren oder/und auf Proteinebene durch Aktivitätsbestimmungen oder immunologische Methoden. Aufgrund der oben gezeigten Korrelation der PLK-Expression in humanen Geweben, primären Zellen und Zellinien mit der Teilungsaktivität der Zellen stellt die Expressionsstärke des PLK-Gens eine geeignete Meßgröße dar, um den Status von bestimmten Zellen hinsichtlich einer Teilungsaktivität zu ermitteln. Dieses extrakorporal durchführbare Verfahren kann z.B. auf dem Gebiet der Tumor- oder Immundiagnostik von großem Nutzen sein.

Die in SEQ ID NO: 1 und 2 dargestellte Aminosäuresequenz stellt das gesamte PLK-Protein dar. Dieses Protein weist 603 Aminosäuren auf. Das PLK-Gen oder Varianten davon können routinemäßig in einem Vektor kloniert werden, so daß dieser Vektor in einer geeigneten Wirtszelle zur Expression gebracht wird, wobei das erfindungsgemäße Protein entsteht. Bevorzugte Wirtszellen sind Mikroorganismen wie E.coli oder Hefe, aber auch höhere Zellen (z.B. Säuger- oder Insektenzellen). Bevorzugte Expressionsvektoren sind z.B. Plasmide, Bakteriophage Lambda für Prokaryonten, Hefevektoren oder virale Vektoren für höhere Zellen (z.B. SV40, Vaccinia, Baculoviren). Bezüglich der Expression des PLK-Gens soll insbesondere auf die bei Sambrook et al. (A Laboratory Manual (1989) Cold Spring Harbor Laboratory Press) genannten Methoden verwiesen werden. Weiterhin ist die Expression verwandter Kinase-Proteine in den Arbeiten von Crews, C.M. et al., (1991), Proc.Natl.Acad.Sci. USA 88, 8845-8849; Ben-David, Y. et al. (1991) EMBO J. 10 (2), 317-325; Parker, L.L. et al. (1991) EMBO J. 10 (5), 1255-1263; Parker L.L. et al. (1992) Science 257, 1955-1957; Colemann, T.R. et al. (1993) Cell 72, 919-929 beschrieben, auf deren Offenbarung hier zu diesem Zweck Bezug genommen wird.

Ein Vergleich der PLK-Sequenz mit der EMBL-Datenbank ergibt ein hohes Maß an Homologie mit der Familie der Serin/Threonin-Kinasen. Das in erfindungsgemäßen Arzneimitteln bzw. diagnostischen Mitteln enthaltene Protein wird aufgrund seiner Homologie zum polo-Gen, das aus Drosophila melanogaster isoliert wurde, als PLK (polo-like-kinase) bezeichnet.

Es treten folgende Protein-Kinase spezifische Sequenzcharakteristika auf:
1. Das ATP-Bindungsmotiv:
GlyLysGlyGlyPheAla ......(16 Aminosäuren) .... Lys (Aminosäuren 60 - 86)
2. Zwei in der katalytischen Domäne von Protein-Kinasen liegende Aminosäure-Motive, die bei allen Protein-Kinasen hinsichtlich der Aminosäuresequenz und des Abstandes der Motive zueinander hoch konserviert sind:

| | |
|---|---|
| HisArgAspLeu | (AS 174 - 177) |
| AspPheGly | (AS 194 - 196) |

Weiterhin ein Gegenstand der vorliegenden Erfindung ist ein Arzneimittel bzw. ein diagnostisches Mittel, das eine Nukleinsäure, die für ein erfindungsgemäß im Arzneimittel bzw. diagnostischen Mittel enthaltenes PLK-Protein oder Teile davon kodiert, enthält. Diese Nukleinsäure kann z.B. genomische DNA oder RNA sein. Vorzugsweise handelt es sich dabei jedoch um ein rekombinantes DNA-Molekül.

Weiterhin ein Gegenstand der Erfindung ist ein Arzneimittel bzw. diagnostisches Mittel, das eine Nukleinsäure enthält, welche
(a) die in SEQ ID NO: 1 dargestellte kodierende Sequenz,
(b) eine der Sequenz aus (a) im Rahmen der Degeneration des genetischen Codes entsprechende Nukleinsäuresequenz oder
(c) eine mit den Sequenzen aus (a) oder/und (b) unter stringenten Hybridisierungsbedingungen hybridisierende Sequenz enthält.

Unter stringenten Hybridisierungsbedingungen im Sinne der vorliegenden Erfindung versteht man, daß eine Hybridisierung auch nach Waschen bei 55°C, vorzugsweise bei 62°C, besonders bevorzugt bei 68°C in einem wäßrigen Niedrigsalz-Puffer (z.B. 0,2 x SSC, 0,1 % SDS) noch auftritt (siehe auch Sambrook, J. et al. (1989), Molecular Cloning. A Laboratory Manual).

Die Erfindung betrifft auch Arzneimittel bzw. diagnostische Mittel, die Nukleinsäuren, die einen mindestens 20 Nukleotide langen Abschnitt der in SEQ ID NO: 1 dargestellten Sequenz enthalten, enthalten. Vorzugsweise besitzt dieser Abschnitt eine für das PLK-Gen spezifische Nukleotidsequenz. Diese Nukleinsäuren eignen sich insbesondere zur Herstellung von therapeutisch einsetzbaren Antisense-Nukleinsäuren.

Noch ein weiterer Gegenstand der Erfindung ist ein Arzneimittel bzw. diagnostisches Mittel, das einen Vektor, der mindestens eine Kopie einer erfindungsgemäß im Arzneimittel enthaltenen Nukleinsäure oder einen Teil davon enthält, enthält. Der Vektor kann in Eukaryonten oder Prokaryonten replizierbar sein. Er kann ein in das Genom der Wirtszelle integrierbarer Vektor (z.B. Bakteriophage Lambda) oder ein Vektor sein, der extrachromosomal vorliegt (z.B. ein Plasmid). Der Vektor kann durch Subklonierung des PLK-Gens in einen Basisvektor erhalten werden. Derartige Basisvektoren, insbesondere Vektoren mit den zur Proteinexpression erforderlichen Elementen sind einem Fachmann geläufig.

Ein weiterer Gegenstand der vorliegenden Erfindung ist ein Arzneimittel bzw. diagnostisches Mittel, das eine Zelle, die mit einer o.g. Nukleinsäure oder einem o.g. Vektor transformiert ist, enthält. Die Zelle kann sowohl eine eukaryontische als auch eine prokaryontische Zelle sein. Verfahren zur Transformation von Zellen sind allgemeiner Stand der Technik und brauchen daher nicht erläutert zu werden.

Die Hemmung des Wachstums von Zellen durch Hemmung der PLK-Expression konnte experimentell durch Antisense-Konstrukte und durch dominant-negative Mutanten gezeigt werden. Ein besonders bevorzugter Aspekt der vorliegenden Erfindung ist daher die gentherapeutische Anwendung der erfindungsgemäßen Nukleinsäuren, bei der diese Nukleinsäuren einem Patienten in replizierbarer Form (z.B. auf einem zur Integration ins Genom geeigneten Vektor) verabreicht werden, z.B. um innerhalb der Zelle Antisense-Nukleinsäuren zu erzeugen. Alternativ können die Antisense-Nukleinsäuren auch direkt (z.B. durch Mikroinjektion) in die Zelle eingeführt werden.

Schließlich sind das PLK-Protein oder Fragmente dieses Proteins geeignet zur Verwendung als Immunogen zur Herstellung von Antikörpern. Die Herstellung von Antikörpern gegen das PLK-Protein erfolgt auf übliche Weise durch Immunisierung von Versuchstieren mit dem vollständigen PLK-Protein oder Fragmenten davon und anschließende Gewinnung der resultierenden (polyklonalen) Antikörper. Nach der Methode von Köhler und Milstein oder deren Weiterentwicklungen können aus den PLK-Antikörper-produzierenden Zellen der Versuchstiere auf bekannte Weise durch Zellfusion monoklonale Antikörper erhalten werden. Ebenso können humane monoklonale Antikörper erhalten werden.

SEQ ID NO:7 zeigt eine 2503 bp lange Nukleinsäuresequenz, die den sich direkt 5'-seitig an das Startcodon des PLK-Gens anschließenden Bereich enthält. Durch Chloramphenicoltransferase-(CAT)-Assays konnte nachgewiesen werden, daß die angegebene Sequenz den Promotor des PLK-Gens beinhaltet. Der Startpunkt der Transkription in SEQ. ID. N0: 7 ist bei Nukleotid 2460. Positiv-regulatorische Elemente des Promotors finden sich innerhalb der Bereiche zwischen den Nukleotiden 184 und 676 bzw. 1163 und 2503. Negativ-regulatorische Elemente finden sich innerhalb des Bereichs zwischen den Nukleotiden 676 und 1163. Diese regulatorischen Elemente konnten mit Hilfe von CAT-Assays mit Deletionsklonen identifiziert werden. Durch Sequenzvergleich wurde weiterhin festgestellt, daß eine CCAAT-Box zwischen den Nukleotiden 2407 und 2411 und zwei potentielle SP1-Bindungsstellen zwischen den Nukleotiden 2375-2384 lokalisiert sind.

Der PLK-Promotor hat die Eigenschaft, daß er nur in proliferierenden Zellen aktiv ist. Er kann daher in Kombination mit einem Gen, das toxisch für die Zellen ist (z.B. dem Gen für das Diphterietoxin oder dem Gen für das Choleratoxin) auch für die gentherapeutische Behandlung von Tumoren eingesetzt werden. Eine weitere therapeutische Einsatzmöglichkeit bietet der PLK-Promotor in Kombination mit dem PLK-Gen in Antisense-Orientierung. Auf diese Weise kann eine selektive Hemmung des Wachstums von sich teilenden Zellen erreicht werden.

Erfindungsgemäß einsetzbar ist somit auch eine Nukleinsäure, welche die in SEQ ID NO:7 gezeigte Sequenz oder einen Abschnitt davon, vorzugsweise einen Abschnitt mit gleicher biologischer Aktivität, enthält. Diese Nukleinsäure kann z.B. in operativer Verknüpfung mit einem toxischen Gen oder mit dem PLK-Gen in Antisense-Orientierung oder einem Abschnitt davon, der vorzugsweise länger als 20 Nukleotide ist, sein. Diese Nukleinsäuren können insbesondere zur Gentherapie eingesetzt werden.

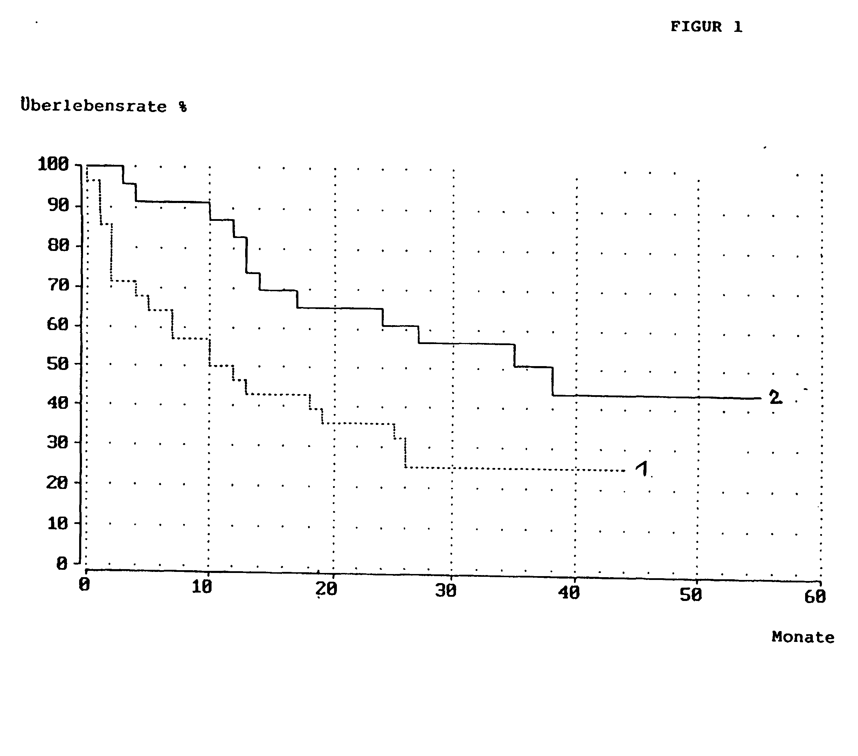

Die Erfindung soll weiter durch die Sequenzprotokolle und Figuren SEQ ID NO:1 bis SEQ ID NO:7 und Fig. 1 zusammen mit den folgenden Beispielen erläutert werden, ohne daß dabei der Umfang der Erfindung beschränkt werden soll.

Es zeigen:
- SEQ ID NO:1: eine 2124 bp lange Nukleinsäuresequenz, die die für das PLK-Gen kodierende genetische Information enthält,
- SEQ ID NO:2: die 603 AS lange Aminosäuresequenz des PLK-Proteins,
- SEQ ID NO:3: die Nukleinsäuresequenz des Oligonukleotidprimers P6 DEA
- SEQ ID NO:4: die Nukleinsäuresequenz des Oligonukleotidprimers Eco HRDL
- SEQ ID NO:5: die Nukleinsäuresequenz des Oligonukleotidprimers P12T+,
- SEQ ID NO:6: die Nukleinsäuresequenz des Oligonukleotidprimers RAF2 und
- SEQ ID NO:7: die 2503 bp lange Nukleinsäuresequenz des PLK-Promotors und
- Figur 1: die postoperative Überlebensrate von Lungenkrebspatienten in Abhängigkeit der PLK-Expression.

### Beispiel 1

### Isolierung einer Teilsequenz des PLK-Gens

1.1 Es wurde gemäß dem Verfahren von Chirgwin et al. (Biochemistry 18 (1979), 5294) RNA aus humanem Lungentumorgewebe isoliert. Mit dieser RNA wurde eine Oligo(dT)-vermittelte cDNA-Synthese durchgeführt. Die Reaktionsbedingungen waren wie folgt:
   50 mmol/l Tris/HCl, pH 8,3 (22°C)
   75 mmol/l KCl
   10 mmol/l Dithiothreitol (DTT)
   3 mmol/l MgCl2
   500 µmol/l dNTP-Lösung
   0,2 µmol/l Oligonukleotid-Primer P12T+ mit der in SEQ ID NO:5 dargestellten Nukleinsäuresequenz
   3,0 µg Gesamt-RNA
   100 µg/ml Rinderserumalbumin
   10 Einheiten Reverse Transkriptase (aus Moloney - Murine Leukemia virus)

   Das obige Reaktionsgemisch wurde in 20 µl Reaktionsvolumen eine Stunde lang bei 37°C inkubiert.
1.2 Die Amplifizierung der cDNA aus 1.1 erfolgte mit zwei zu hoch konservierten Regionen von PTK-Genen komplementären Primern Eco HRDL und P6 DEA. Die Nukleinsäuresequenz des Primers P6 DEA ist in SEQ ID NO:3 angegeben. Die Sequenz von Eco HRDL ist in SEQ ID NO:4 dargestellt (diese Sequenz ist den Nukleotiden 588 bis 601 der in SEQ ID NO:1 dargestellten Sequenz homolog).

### Reaktionsbedingungen:

8,3 mmol/l Tris/HCl, pH 8,8 (22°C)
41,7 mmol/l KCl
1,25 mmol/l MgCl2
0,01 % Gelatine
166,7 µmol/l dNTP-Lösung
0,6 µmol/l Primer P6 DEA und Eco HRDL
5 Einheiten Taq-Polymerase
10 µl cDNA-Syntheseansatz

Das Reaktionsvolumen war 60 µl. Die Bedingungen für einen PCR-Zyklus waren wie folgt:
95°C Denaturierung für 1 Minute,
40°C Hybridisierung für 2 Minuten,
72°C Elongation für 3 Minuten.
Es wurden insgesamt 40 Zyklen durchgeführt.

Das gebildete Amplifikat wurde zwecks Aufreinigung in einem 2 %igen Agarosegel elektrophoretisch aufgetrennt und die DNA im Längenbereich von ca. 190 bp bis 220 bp elektrisch eluiert und erneut unter den oben angegebenen Bedingungen für 30 Zyklen amplifiziert. Die so erhaltenen und gereinigten PCR-Produkte wurden anschließend mit der Restriktionsendonuklease EcoRI verdaut und in den ebenfalls mit EcoRI gespaltenen Vektor Bluescript-KS (Stratagene) kloniert.

Auf diese Weise wurde ein 199 bp langes DNA-Fragment isoliert und sequenziert (Sambrook, J. et al., Molecular Cloning, A Laboratory Manual, Cold Spring Harbor Laboratory 1989). Die Nukleinsäuresequenz dieses DNA-Fragments entspricht den Nukleotiden 588 bis 787 der in SEQ ID NO:1 dargestellten Sequenz.

### Beispiel 2

### Isolierung einer PLK-cDNA

Es wurde eine cDNA-Bank aus poly A+ RNA von humanem Lungentumorgewebe mit 1,8 x 106 rekombinanten Klonen etabliert. Die Isolierung der mRNA erfolgte gemäß Aviv und Leder (Proc.Natl. Acad.Sci. USA 69 (1972), 1408). Die cDNA-Klonierung erfolgte gemäß Gubler und Hoffman (Gene 25 (1983), 263). Diese Genbank wurde mittels einer DNA-Sonde durchmustert. Die DNA-Sonde wurde durch PCR hergestellt. Als Primer für die PCR wurde das Oligonukleotid RAF2 verwendet. Die Nukleinsäuresequenz für RAF2 ist in SEQ ID NO:6 dargestellt (diese Sequenz entspricht den Nukleotiden 746 bis 767 der in SEQ ID NO:1 dargestellten Sequenz).

### Reaktionsbedingungen:

20 ng PLK Insert (aus Beispiel 1)
10 mmol/l Tris/HCl, pH 8,8 (22°C)
50 mmol/l KCl
1,5 mmol/l MgCl2
0,01 % Gelatine
0,8 µmol/l α32P-dCTP (6000 Ci/mmol)
0,8 µmol/l dATP/dGTP/dTTP
0,2 µmol/l des Primers RAF2
2,5 Einheiten Taq-Polymerase

Das Reaktionsvolumen war 25 µl. Die Durchführung der Reaktion erfolgte in 20 Zyklen bei den oben (in Beispiel 1) genannten PCR-Bedingungen.

Zum Nachweis eines positiven Klons wurde cDNA aus der Genbank auf Filtern immobilisiert und an die radioaktiv markierte DNA-Sonde (1 x 106 dpm/ml Lösung bei einer spezifischen Sondenaktivität von 5 x 109 dpm/µg DNA) hybridisiert. Die Hybridisierungstemperatur war 42°C.

### Hybridisierungslösung:

5 x SSC
0,02 mol/l Tris/HCl, pH 7,6 (22°C)
1 x Denhardt-Lösung
10 % Dextransulfat
0,1 % SDS
100 µg/ml Heringssperma DNA
50 % Formamid

Als Waschlösung wurde 2 x SSC, 0,1 % SDS bei einer Waschtemperatur von 42°C eingesetzt.

Es wurde ein positiver Klon gefunden und charakterisiert. Er enthielt die in SEQ ID NO:1 dargestellte Nukleinsäuresequenz.

### Beispiel 3

### Hemmung der PLK-Expression

1 x 10⁴ A431 Zellen wurden ausgesät und für 2 bis 3 Tage zu einer Konfluenz von 95 % wachsen gelassen. Dann wurde die Kultur in RPMI 1640 Medium mit 0,5 % fetalem Kälberserum für 24 Stunden gehalten. Pro Zelle wurden durch Mikroinjektion 1 bis 2 x 10⁻¹² ml einer Flüssigkeit mit 2 µg/µl TKF-Antisense RNA injiziert. Diese TKF-Antisense-RNA wurde durch Transkription der in SEQ ID N0: 1 gezeigten Sequenz in umgekehrter Orientierung mit T3 oder T7 RNA-Polymerase hergestellt.

Die Zellen wurden weiter in RPMI 1640 Medium mit 0,5 % oder 10 % fetalem Kälberserum gehalten. Nach 18 Stunden wurden die Zellen mit ³H-Thymidin (0,5 µCi/ml; Amersham) für 3 bis 4 Stunden gepulst. Nach Waschen mit Phosphor-gepufferter Salzlösung (PBS) erfolgte eine Fixierung in 3,5 % Formaldehyd-PBS, dann eine Überschichtung mit Filmemulsion (NTP-2, Kodak) und eine Inkubation für 48 Stunden zum Zweck der Entwicklung. Die Zellen wurden nach Giemsa gefärbt, gezählt und fotografiert. Es wurde gezeigt, daß durch Zugabe der Antisense-RNA die PLK-Expression und das Wachstum von Zellen gehemmt werden konnte.

### Beispiel 4

### PLK als diagnostischer Tumormarker

Es wurde festgestellt, daß bei operierten Lungentumorpatienten eine eindeutige Korrelation der PLK-Expression mit der Überlebensdauer des Patienten besteht. Es wurde gefunden, daß Patienten mit hoher PLK-Expression wesentlich früher sterben als die in der Vergleichsgruppe mit geringer PLK-Expression.

Die Ergebnisse dieses Experiments sind in der folgenden Tabelle 2 angegeben. Zur Analyse der PLK-Expression wurde die mRNA aus dem Tumorgewebe des jeweiligen Patienten mittels einer Northern-Blot-Analyse untersucht. Die in Tabelle 2 angegebenen Werte sind bezüglich der Expression von Aktin mRNA standardisiert. Die Überlebensdauer der Patienten nach der Operation ist in Monaten angegeben. Das Symbol "*" bedeutet, daß der Patient zum angegebenen Zeitpunkt noch lebte.

**Tabelle 2**

| Patient Nr. | Alter/ Geschlecht | Tumorstadium | Tumor | post-operative Überlebensdauer | PLK Expression |
|---|---|---|---|---|---|
| | | | Plattenepithelzellkarzinom | | |
| 1 | 71m | II | | 10 | 3,79 |
| 2 | 71m | III | | *28 | 0,48 |
| 3 | 60m | III | | 5 | 1,02 |
| 4 | 54m | III | | 19 | 1,12 |
| 5 | 61m | I | | 17 | 0,36 |
| 6 | 67m | ? | | 24 | 0,15 |
| 7 | 70m | I | | 35 | 0,90 |
| 8 | 69m | II | | *41 | 0,08 |
| 9 | 72m | I | | *37 | 0,67 |
| 10 | 67m | III | | *55 | 0,35 |
| 11 | 66m | II | | 10 | 0,13 |
| 12 | 57m | I | | *31 | 0,10 |
| 13 | 61m | I | | 4 | 6,94 |
| 14 | 64m | I | | 7 | 1,96 |
| 15 | 42m | II | | 27 | 0,28 |
| 16 | 70m | I | | 0 | 3,84 |
| 17 | 54m | II | | 25 | 4,66 |
| 18 | 61m | II | | 12 | 1,99 |
| 19 | 67m | I | | 7 | 1,14 |
| 20 | 70m | II | | 0 | 3,75 |
| 21 | 53f | I | | *38 | 1,41 |
| 22 | 36m | II | | *37 | 8,57 |
| 23 | 75m | I | | 4 | 2,01 |
| | | | Adenokarzinom | | |
| 24 | 77f | I | | *38 | 0,31 |
| 25 | 56m | II | | *32 | 0,73 |
| 26 | 56m | III | | 13 | 2,14 |
| 27 | 71m | III | | 2 | 13,1 |
| 28 | 72m | I | | *32 | 1,04 |
| 29 | 54m | III | | 14 | 0,94 |
| 30 | 60m | I | | 26 | 1,34 |
| 31 | 70m | I | | 38 | 0,96 |
| 32 | 43m | I | | *32 | 4,12 |
| | | | Großzelliges Karzinom | | |
| 33 | 62m | III | | 26 | 3,97 |
| 34 | 66m | I | | *44 | 14,8 |
| 35 | 66f | III | | 2 | 32,3 |
| 36 | 64m | I | | 2 | 11,0 |
| 37 | 40m | I | | *36 | 17,9 |
| 38 | 53f | III | | 0 | 5,50 |
| | | | Gemischtzelliges Karzinom | | |
| 39 | 61m | I | | 4 | 0,10 |
| 40 | 57m | II | | 0 | 3,34 |
| 41 | 49m | I | | 3 | 0,33 |
| | | | Broncheoalveolares Karzinom | | |
| 42 | 76f | I | | *43 | 0,40 |
| 43 | 77f | I | | *44 | 0,12 |
| 44 | 77f | II | | *48 | 0,02 |
| 45 | 57m | I | | *42 | 0,12 |
| | | | Kleinzelliger Lungenkrebs | | |
| 46 | 77m | III | | 13 | 0,55 |
| 47 | 62m | I | | 10 | 6,23 |
| 48 | 63m | III | | 18 | 11,2 |
| 49 | 58m | I | | *35 | 11,2 |
| 50 | 63m | II | | 13 | 0,04 |
| | | | Leiomyosarkom in der Lunge | | |
| 51 | 46m | III | | 12 | 0,42 |

Fig. 1 zeigt die zugehörige statistische Kaplan-Meier-Auswertung der Patientendaten (E.L. Kaplan und P. Meier, J. Am. Stat. Assoc. 53 (1958), 457-481). Kurve 1 zeigt die postoperative Überlebensrate von 23 Lungenkrebspatienten mit einer absoluten PLK-Expression > 1 (standardisiert auf Aktin). Kurve 2 zeigt die Überlebensrate von 28 Lungenkrebspatienten mit einer absoluten standardisierten PLK-Expression < 1. Aus Fig. 1 wird ersichtlich, daß Patienten mit hoher PLK-Expression wesentlich früher sterben als die in der Vergleichsgruppe mit geringer PLK-Expression.

## Patentansprüche

1. Arzneimittel zur Verwendung bei der Tumortherapie,
**dadurch gekennzeichnet,**
**dass** es als aktiven Bestandteil ein PLK-Protein enthält, welches
(a) die in SEQ ID NO.2 dargestellte Aminosäuresequenz oder
(b) Teile oder/und Varianten der Sequenz aus (a), welche auf Aminosäureebene eine Homologie von mindestens 95 % gegenüber der Sequenz aus (a) aufweisen und hinsichtlich ihrer biologischen oder/und immunologischen Aktivität dem in SEQ ID NO.1 dargestellten Protein entsprechen, umfasst,
oder einen gegen ein solches PLK-Protein gerichteten Antikörper oder eine Nukleinsäure, welche für ein solches PLK-Protein codiert, gegebenenfalls zusammen mit pharmazeutisch üblichen Hilfs-, Träger-, Füll- oder Verdünnungsmitteln.

2. Diagnostisches Mittel zur Verwendung bei der Tumordiagnostik oder bei der Bestimmung der Aktivität von Lymphozyten,
**dadurch gekennzeichnet,**
**dass** es als aktiven Bestandteil ein PLK-Protein, welches
(a) die in SEQ ID NO.2 dargestellte Aminosäuresequenz oder
(b) Teile oder/und Varianten der Sequenz aus (a), welche auf Aminosäureebene eine Homologie von mindestens 95 % gegenüber der Sequenz aus (a) aufweisen und hinsichtlich ihrer biologischen oder/und immunologischen Aktivität dem in SEQ ID NO.1 dargestellten Protein entsprechen, umfasst,
einen Antikörper gegen ein solches PLK-Protein oder eine Nukleinsäure, welche für ein solches PLK-Protein codiert, enthält.

3. Arzneimittel bzw. diagnostisches Mittel gemäß einem der Ansprüche 1 oder 2,
**dadurch gekennzeichnet,**
**dass** es ein PLK-Protein enthält, welches aus dem Menschen erhältlich ist.

4. Arzneimittel bzw. diagnostisches Mittel nach einem der Ansprüche 1 bis 3,
**dadurch gekennzeichnet,**
**dass** es eine Nukleinsäure enthält, welche ein rekombinantes DNA-Molekül ist.

5. Arzneimittel bzw. diagnostisches Mittel nach einem der vorhergehenden Ansprüche,
**dadurch gekennzeichnet,**
**dass** es eine Nukleinsäure umfasst, welche
(a) die in SEQ ID NO.1 dargestellte Protein-codierende Sequenz,
(b) eine der Sequenz aus (a) im Rahmen der Degeneration des genetischen Codes entsprechende Nukleinsäuresequenz oder
(c) eine mit den Sequenzen aus (a) oder/und (b) unter stringenten Hybridisierungsbedingungen hybridisierende Sequenz enthält.

6. Arzneimittel bzw. diagnostisches Mittel nach einem der vorhergehenden Ansprüche,
**dadurch gekennzeichnet,**
**dass** es eine Nukleinsäure enthält, die einen mindestens 20 Nukleotide langen Abschnitt der in SEQ ID NO.1 dargestellten Sequenz enthält.

7. Arzneimittel bzw. diagnostisches Mittel nach einem der Ansprüche 1 bis 6 zur Bestimmung der Lymphozytenaktivität bei Störungen des Immunsystems, insbesondere bei Autoimmunerkrankungen oder Immunmangelsyndromen.

8. Arzneimittel bzw. diagnostisches Mittel nach einem der Ansprüche 1 bis 6 zur Verwendung in der Gentherapie.

## Claims

1. Pharmaceutical composition for use in tumour therapy, **characterised in that**, as an active constituent, it contains a PLK protein which
(a) comprises the amino acid sequence shown in SEQ ID NO. 2 or
(b) parts or/and variants of the sequence from (a) which, on an amino acid level, have a homology of at least 95% with respect to the sequence from (a) and correspond with respect to their biological or/and immunological activity to the protein shown in SEQ ID NO.1,
or an antibody directed against a PLK protein of this type or a nucleic acid which codes for a PLK protein of this type, optionally together with pharmaceutically conventional auxiliaries, excipients, fillers or diluents.

2. Diagnostic composition for use in tumour diagnosis or in determining the activity of lymphocytes, **characterised in that**, as an active constituent, it contains a PLK protein which
(a) comprises the amino acid sequence shown in SEQ ID NO. 2 or
(b) parts or/and variants of the sequence from (a) which, on an amino acid level, have a homology of at least 95% with respect to the sequence from (a) and correspond with respect to their biological or/and immunological activity to the protein shown in SEQ ID NO. 1,
an antibody against a PLK protein of this type or a nucleic acid which codes for a PLK protein of this type.

3. Pharmaceutical composition or diagnostic composition according to either Claim 1 or Claim 2, **characterised in that** it contains a PLK protein which can be obtained from humans.

4. Pharmaceutical composition or diagnostic composition according to any one of Claims 1 to 3, **characterised in that** it contains a nucleic acid which is a recombinant DNA molecule.

5. Pharmaceutical composition or diagnostic composition according to any one of the preceding claims, **characterised in that** it comprises a nucleic acid which
(a) contains the protein-coding sequence shown in SEQ ID NO.1,
(b) a nucleic acid sequence corresponding to the sequence from (a) in the course of the degeneration of the genetic code or
(c) a sequence hybridising with the sequences from (a) or/and (b) under stringent hybridising conditions.

6. Pharmaceutical composition or diagnostic composition according to any one of the preceding claims, **characterised in that** it contains a nucleic acid which contains a section, which is at least 20 nucleotides long, of the sequence shown in SEQ ID NO. 1.

7. Pharmaceutical composition or diagnostic composition according to any one of Claims 1 to 6 for determining the lymphocyte activity in the event of disorders of the immune system, in particular in autoimmune disorders or immunodeficiency syndromes.

8. Pharmaceutical composition or diagnostic composition according to any one of Claims 1 to 6 for use in gene therapy.

## Revendications

1. Médicament à utiliser dans la thérapie des tumeurs, **caractérisé en ce qu'**il contient comme constituant actif une protéine PLK, qui
(a) comprend la séquence d'aminoacides représentée par SEQ ID NO. 2 ou
(b) comprend des parties et/ou des variantes de la séquence de (a), qui présentent sur le plan des aminoacides une homologie d'au moins 95 % avec la séquence de (a) et qui, en ce qui concerne leur activité biologique et/ou immunologique, correspondent à la protéine représentée par SEQ ID NO. 1,
ou un anticorps dirigé contre une telle protéine PLK, ou un acide nucléique qui code pour une telle protéine PLK, éventuellement avec des agents auxiliaires, des supports, des charges ou des diluants classiques en pharmacie.

2. Agent diagnostique à utiliser dans le diagnostic des tumeurs ou dans la détermination de l'activité de lymphocytes, **caractérisé en ce qu'**il contient comme constituant actif une protéine PLK, qui
(a) comprend la séquence d'aminoacides représentée par SEQ ID NO. 2 ou
(b) comprend des parties et/ou des variantes de la séquence de (a), qui présentent sur le plan des aminoacides une homologie d'au moins 95 % avec la séquence de (a) et qui, en ce qui concerne leur activité biologique et/ou immunologique, correspondent à la protéine représentée par SEQ ID NO. 1,
un anticorps dirigé contre une telle protéine PLK, ou un acide nucléique qui code pour une telle protéine PLK.

3. Médicament ou agent diagnostique selon l'une des revendications 1 ou 2, **caractérisé en ce qu'**il contient une protéine PLK qui peut être obtenue à partir de l'être humain.

4. Médicament ou agent diagnostique selon l'une des revendications 1 à 3, **caractérisé en ce qu'**il contient un acide nucléique qui est une molécule d'ADN recombiné.

5. Médicament ou agent diagnostique selon l'une des revendications précédentes, **caractérisé en ce qu'**il comprend un acide nucléique qui
(a) contient la séquence codant pour la protéine représentée par SEQ ID NO. 1,
(b) contient une séquence d'acide nucléique correspondant à la séquence de (a) dans le cadre de la dégénérescence du code génétique ou
(c) contient une séquence s'hybridant avec les séquences de (a) et/ou (b) dans des conditions d'hybridation stringentes.

6. Médicament ou agent diagnostique selon l'une des revendications précédentes, **caractérisé en ce qu'**il contient un acide nucléique qui contient un fragment d'au moins 20 nucléotides de la séquence représentée par SEQ ID NO. 1.

7. Médicament ou agent diagnostique selon l'une des revendications 1 à 6 destiné à la détermination de l'activité des lymphocytes en cas de troubles du système immunitaire, en particulier de maladies auto-immunes ou du syndrome de déficit immunitaire.

8. Médicament ou agent diagnostique selon l'une des revendications 1 à 6 à utiliser dans la thérapie génique.


### SEQUENZPROTOKOLL

(1) ALGEMEINE INFORMATION:
   (i) ANMELDER:
      (A) NAME: Chemotherapeutisches Forschungsinstitut Georg-Speyer-Haus
      (B) STRASSE: Paul-Ehrlich-Strasse 42-44
      (C) ORT: Frankfurt
      (E) LAND: Deutschland
      (F) POSTLEITZAHL: 60596
   (ii) ANMELDETITEL: Klonierung eines neuen Mitgliedes der Familie der Serin-Threonin-Kinasen
   (iii) ANZAHL DER SEQUENZEN: 7
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 2124 Basenpaare
      (B) ART: Nukleinsäure
      (C) STRANGFORM: beides
      (D) TOPOLOGIE: linear
   (ii) ART DES MOLEKÜLS: cDNS
   (iii) ANTISENSE: JA
   (vi) URSPRÜNLICHE HERKUNFT:
      (A) ORGANISMUS: Homo sapiens
   (ix) MERKMALE:
      (A) NAME/SCHLÜSSEL: CDS
      (B) LAGE: 65..1873
   (xi) SEQUENZBESCHREIBUNG: SEQ ID NO: 1:
   GGGGAGGAGC GGAGCGGTGC GGAGGCTCTG CTCGGATCGA GGTCTGCAGC GCAGCTTCGG        60
   GAGC ATG AGT GCT GCA GTG ACT GCA GGG AAG CTG GCA CGG GCA CCG GCC       109
        Met Ser Ala Ala Val Thr Ala Gly Lys Leu Ala Arg Ala Pro Ala
         1               5                  10                  15
   GAC CCT GGG AAA GCC GGG GTC CCC GGA GTT GCA GCT CCC GGA GCT CCG        157
   Asp Pro Gly Lys Ala Gly Val Pro Gly Val Ala Ala Pro Gly Ala Pro
                   20                  25                  30
   GCG GCG GCT CCA CCG GCG AAA GAG ATC CCG GAG GTC CTA GTG GAC CCA        205
   Ala Ala Ala Pro Pro Ala Lys Glu Ile Pro Glu Val Leu Val Asp Pro
               35                  40                  45
   CGC AGC CGG CGG CGC TAT GTG CGG GGC CGC TTT TTG GGC AAG GGC GGC        253
   Arg Ser Arg Arg Arg Tyr Val Arg Gly Arg Phe Leu Gly Lys Gly Gly
           50                  55                  60
   TTT GCC AAG TGC TTC GAG ATC TCG GAC GCG GAC ACC AAG GAG GTG TTC        301
   Phe Ala Lys Cys Phe Glu Ile Ser Asp Ala Asp Thr Lys Glu Val Phe
       65                  70                  75
   GCG GGC AAG ATT GTG CCT AAG TCT CTG CTG CTC AAG CCG CAC CAG AGG      349
   Ala Gly Lys Ile Val Pro Lys Ser Leu Leu Leu Lys Pro His Gln Arg
    80                  85                  90                  95
   GAG AAG ATG TCC ATG GAA ATA TCC ATT CAC CGC AGC CTC GCC CAC CAG      397
   Glu Lys Met Ser Met Glu Ile Ser Ile His Arg Ser Leu Ala His Gln
               100                 105                 110
   CAC GTC GTA GGA TTC CAC GGC TTT TTC GAG GAC AAC GAC TTC GTG TTC      445
   His Val Val Gly Phe His Gly Phe Phe Glu Asp Asn Asp Phe Val Phe
               115                 120                 125
   GTG GTG TTG GAG CTC TGC CGC CGG AGG TCT CTC CTG GAG CCG CAC AAG      493
   Val Val Leu Glu Leu Cys Arg Arg Arg Ser Leu Leu Glu Pro His Lys
           130                 135                 140
   AGG AGG AAA GCC CTG ACT GAG CCT GAG GCC CGA TAC TAC CTA CGG CAA      541
   Arg Arg Lys Ala Leu Thr Glu Pro Glu Ala Arg Tyr Tyr Leu Arg Gln
       145                 150                 155
   ATT GTG CTT GGC TGC CAG TAC CTG CAC CGA AAC CGA GTT ATT CAT CGA      589
   Ile Val Leu Gly Cys Gln Tyr Leu His Arg Asn Arg Val Ile His Arg
   160                 165                 170                 175
   GAC CTC AAG CTG GGC AAC CTT TTC CTG AAT GAA GAT CTG GAG GTG AAA      637
   Asp Leu Lys Leu Gly Asn Leu Phe Leu Asn Glu Asp Leu Glu Val Lys
               180                 185                 190
   ATA GGG GAT TTT GGA CTG GCA ACC AAA GTC GAA TAT GAC GGG GAG AGG      685
   Ile Gly Asp Phe Gly Leu Ala Thr Lys Val Glu Tyr Asp Gly Glu Arg
           195                 200                 205
   AAG AAG ACC CTG TGT GGG ACT CCT AAT TAC ATA GCT CCC GAG GTG CTG      733
   Lys Lys Thr Leu Cys Gly Thr Pro Asn Tyr Ile Ala Pro Glu Val Leu
           210                 215                 220
   AGC AAG AAA GAG CAC AGT TTC GAG GTG GAT GTG TGG TCC ATT GGG TGT      781
   Ser Lys Lys Glu His Ser Phe Glu Val Asp Val Trp Ser Ile Gly Cys
       225                 230                 235
   ATC ATG TAT ACC TTG TTA GTG GGC AAA CCA CCT TTT GAG ACT TCT TGC      829
   Ile Met Tyr Thr Leu Leu Val Gly Lys Pro Pro Phe Glu Thr Ser Cys
   240                 245                 250                 255
   CTA AAA GAG ACC TAC CTC CGG ATC AAG AAG AAT GAA TAC AGT ATT CCC      877
   Leu Lys Glu Thr Tyr Leu Arg Ile Lys Lys Asn Glu Tyr Ser Ile Pro
               260                 265                 270
   AAG CAC ATC AAC CCC GTG GCC GCC TCC CTC ATC CAG AAG ATG CTT CAG      925
   Lys His Ile Asn Pro Val Ala Ala Ser Leu Ile Gln Lys Met Leu Gln
           275                 280                 285
   ACA GAT CCC ACT GCC CGC CCA ACC ATT AAC GAG CTG CTT AAT GAC GAG      973
   Thr Asp Pro Thr Ala Arg Pro Thr Ile Asn Glu Leu Leu Asn Asp Glu
           290                 295                 300
   TTC TTT ACT TCT GGC TAT ATC CCT GCC CGT CTC CCC ATC ACC TGC CTG     1021
   Phe Phe Thr Ser Gly Tyr Ile Pro Ala Arg Leu Pro Ile Thr Cys Leu
       305                 310                 315
   ACC ATT CCA CCA AGG TTT TCG ATT GCT CCC AGC AGC CTG GAC CCC AGC     1069
   Thr Ile Pro Pro Arg Phe Ser Ile Ala Pro Ser Ser Leu Asp Pro Ser
   320                 325                 330                 335
   AAC CGG AAG CCC CTC ACA GTC CTC AAT AAA GGC TTG GAG AAC CCC CTG     1117
   Asn Arg Lys Pro Leu Thr Val Leu Asn Lys Gly Leu Glu Asn Pro Leu
               340                 345                 350
   CCT GAG CGT CCC CGG GAA AAA GAA GAA CCA GTG GTT CGA GAG ACA GGT      1165
   Pro Glu Arg Pro Arg Glu Lys Glu Glu Pro Val Val Arg Glu Thr Gly
               355                 360                 365
   GAG GTG GTC GAC TGC CAC CTC AGT GAC ATG CTG CAG CAG CTG CAC AGT      1213
   Glu Val Val Asp Cys His Leu Ser Asp Met Leu Gln Gln Leu His Ser
           370                 375                 380
   GTC AAT GCC TCC AAG CCC TCG GAG CGT GGG CTG GTC AGG CAA GAG GAG      1261
   Val Asn Ala Ser Lys Pro Ser Glu Arg Gly Leu Val Arg Gln Glu Glu
       385                 390                 395
   GCT GAG GAT CCT GCC TGC ATC CCC ATC TTC TGG GTC AGC AAG TGG GTG      1309
   Ala Glu Asp Pro Ala Cys Ile Pro Ile Phe Trp Val Ser Lys Trp Val
   400                 405                 410                 415
   GAC TAT TCG GAC AAG TAC GGC CTT GGG TAT CAG CTC TGT GAT AAC AGC      1357
   Asp Tyr Ser Asp Lys Tyr Gly Leu Gly Tyr Gln Leu Cys Asp Asn Ser
                   420                 425                 430
   GTG GGG GTG CTC TTC AAT GAC TCA ACA CGC CTC ATC CTC TAC AAT GAT      1405
   Val Gly Val Leu Phe Asn Asp Ser Thr Arg Leu Ile Leu Tyr Asn Asp
               435                 440                 445
   GGT GAC AGC CTG CAG TAC ATA GAG CGT GAC GGC ACT GAG TCC TAC CTC      1453
   Gly Asp Ser Leu Gln Tyr Ile Glu Arg Asp Gly Thr Glu Ser Tyr Leu
           450                 455                 460
   ACC GTG AGT TCC CAT CCC AAC TCC TTG ATG AAG AAG ATC ACC CTC CTT      1501
   Thr Val Ser Ser His Pro Asn Ser Leu Met Lys Lys Ile Thr Leu Leu
       465                 470                 475
   AAA TAT TTC CGC AAT TAC ATG AGC GAG CAC TTG CTG AAG GCA GGT GCC      1549
   Lys Tyr Phe Arg Asn Tyr Met Ser Glu His Leu Leu Lys Ala Gly Ala
   480                 485                 490                 495
   AAC ATC ACG CCG CGC GAA GGT GAT GAG CTC GCC CGG CTG CCC TAC CTA      1597
   Asn Ile Thr Pro Arg Glu Gly Asp Glu Leu Ala Arg Leu Pro Tyr Leu
                   500                 505                 510
   CGG ACC TGG TTC CGC ACC CGC AGC GCC ATC ATC CTG CAC CTC AGC AAC      1645
   Arg Thr Trp Phe Arg Thr Arg Ser Ala Ile Ile Leu His Leu Ser Asn
               515                 520                 525
   GGC AGC GTG CAG ATC AAC TTC TTC CAG GAT CAC ACC AAG CTC ATC TTG      1693
   Gly Ser Val Gln Ile Asn Phe Phe Gln Asp His Thr Lys Leu Ile Leu
           530                 535                 540
   TGC CCA CTG ATG GCA GCC GTG ACC TAC ATC GAC GAG AAG CGG GAC TTC      1741
   Cys Pro Leu Met Ala Ala Val Thr Tyr Ile Asp Glu Lys Arg Asp Phe
       545                 550                 555
   CGC ACA TAC CGC CTG AGT CTC CTG GAG GAG TAC GGC TGC TGC AAG GAG      1789
   Arg Thr Tyr Arg Leu Ser Leu Leu Glu Glu Tyr Gly Cys Cys Lys Glu
   560                 565                 570                 575
   CTG GCC AGC CGG CTC CGC TAC GCC CGC ACT ATG GTG GAC AAG CTG CTG      1837
   Leu Ala Ser Arg Leu Arg Tyr Ala Arg Thr Met Val Asp Lys Leu Leu
                   580                 585                 590
   AGC TCA CGC TCG GCC AGC AAC CGT CTC AAG GCC TCC TAATAGCTGC           1883
   Ser Ser Arg Ser Ala Ser Asn Arg Leu Lys Ala Ser
               595                 600
   CCTCCCCTCC GGACTGGTGC CCTCCTCACT CCCACCTGCA TCTGGGGCCC ATACTGGTTG    1943
   GCTCCCGCGG TGCCATGTCT GCAGTGTGCC CCCCAGCCCC GGTGGCTGGG CAGAGCTGCA    2003
   TCATCCTTGC AGGTGGGGGT TGCTGTATAA GTTATTTTTG TACATGTTCG GGTGTGGGTT    2063
   CTACAGACTT GTCCCCCTCC CCCTCAACCC CACCATATGA ATTGTACAGA ATATTTCTAT    2123
   T                                                                    2124
(2) INFORMATION ZU SEQ ID NO: 2:
   (i) SEQUENZ CHARAKTERISTIKA:
      (A) LÄNGE: 603 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Ser Ala Ala Val Thr Ala Gly Lys Leu Ala Arg Ala Pro Ala Asp
   1               5                   10                  15
   Pro Gly Lys Ala Gly Val Pro Gly Val Ala Ala Pro Gly Ala Pro Ala
               20                  25                  30
   Ala Ala Pro Pro Ala Lys Glu Ile Pro Glu Val Leu Val Asp Pro Arg
           35                  40                  45
   Ser Arg Arg Arg Tyr Val Arg Gly Arg Phe Leu Gly Lys Gly Gly Phe
       50                  55                  60
   Ala Lys Cys Phe Glu Ile Ser Asp Ala Asp Thr Lys Glu Val Phe Ala
   65                  70                  75                  80
   Gly Lys Ile Val Pro Lys Ser Leu Leu Leu Lys Pro His Gln Arg Glu
                   85                  90                  95
   Lys Met Ser Met Glu Ile Ser Ile His Arg Ser Leu Ala His Gln His
               100                 105                 110
   Val Val Gly Phe His Gly Phe Phe Glu Asp Asn Asp Phe Val Phe Val
           115                 120                 125
   Val Leu Glu Leu Cys Arg Arg Arg Ser Leu Leu Glu Pro His Lys Arg
       130                 135                 140
   Arg Lys Ala Leu Thr Glu Pro Glu Ala Arg Tyr Tyr Leu Arg Gln Ile
   145                 150                 155                 160
   Val Leu Gly Cys Gln Tyr Leu His Arg Asn Arg Val Ile His Arg Asp
                   165                 170                 175
   Leu Lys Leu Gly Asn Leu Phe Leu Asn Glu Asp Leu Glu Val Lys Ile
               180                 185                 190
   Gly Asp Phe Gly Leu Ala Thr Lys Val Glu Tyr Asp Gly Glu Arg Lys
           195                 200                 205
   Lys Thr Leu Cys Gly Thr Pro Asn Tyr Ile Ala Pro Glu Val Leu Ser
       210                 215                 220
   Lys Lys Glu His Ser Phe Glu Val Asp Val Trp Ser Ile Gly Cys Ile
   225                 230                 235                 240
   Met Tyr Thr Leu Leu Val Gly Lys Pro Pro Phe Glu Thr Ser Cys Leu
                   245                 250                 255
   Lys Glu Thr Tyr Leu Arg Ile Lys Lys Asn Glu Tyr Ser Ile Pro Lys
                   260                 265                 270
   His Ile Asn Pro Val Ala Ala Ser Leu Ile Gln Lys Met Leu Gln Thr
           275                 280                 285
   Asp Pro Thr Ala Arg Pro Thr Ile Asn Glu Leu Leu Asn Asp Glu Phe
       290                 295                 300
   Phe Thr Ser Gly Tyr Ile Pro Ala Arg Leu Pro Ile Thr Cys Leu Thr
   305                 310                 315                 320
   Ile Pro Pro Arg Phe Ser Ile Ala Pro Ser Ser Leu Asp Pro Ser Asn
                   325                 330                 335
   Arg Lys Pro Leu Thr Val Leu Asn Lys Gly Leu Glu Asn Pro Leu Pro
               340                 345                 350
   Glu Arg Pro Arg Glu Lys Glu Glu Pro Val Val Arg Glu Thr Gly Glu
           355                 360                 365
   Val Val Asp Cys His Leu Ser Asp Met Leu Gln Gln Leu His Ser Val
       370                 375                 380
   Asn Ala Ser Lys Pro Ser Glu Arg Gly Leu Val Arg Gln Glu Glu Ala
   385                 390                 395                 400
   Glu Asp Pro Ala Cys Ile Pro Ile Phe Trp Val Ser Lys Trp Val Asp
                   405                 410                 415
   Tyr Ser Asp Lys Tyr Gly Leu Gly Tyr Gln Leu Cys Asp Asn Ser Val
               420                 425                 430
   Gly Val Leu Phe Asn Asp Ser Thr Arg Leu Ile Leu Tyr Asn Asp Gly
           435                 440                 445
   Asp Ser Leu Gln Tyr Ile Glu Arg Asp Gly Thr Glu Ser Tyr Leu Thr
       450                 455                 460
   Val Ser Ser His Pro Asn Ser Leu Met Lys Lys Ile Thr Leu Leu Lys
   465                 470                 475                 480
   Tyr Phe Arg Asn Tyr Met Ser Glu His Leu Leu Lys Ala Gly Ala Asn
                   485                 490                 495
   Ile Thr Pro Arg Glu Gly Asp Glu Leu Ala Arg Leu Pro Tyr Leu Arg
               500                 505                 510
   Thr Trp Phe Arg Thr Arg Ser Ala Ile Ile Leu His Leu Ser Asn Gly
           515                 520                 525
   Ser Val Gln Ile Asn Phe Phe Gln Asp His Thr Lys Leu Ile Leu Cys
       530                 535                 540
   Pro Leu Met Ala Ala Val Thr Tyr Ile Asp Glu Lys Arg Asp Phe Arg
   545                 550                 555                 560
   Thr Tyr Arg Leu Ser Leu Leu Glu Glu Tyr Gly Cys Cys Lys Glu Leu
                   565                 570                 575
   Ala Ser Arg Leu Arg Tyr Ala Arg Thr Met Val Asp Lys Leu Leu Ser
               580                 585                 590
   Ser Arg Ser Ala Ser Asn Arg Leu Lys Ala Ser
           595                 600
(2) INFORMATION ZU SEQ ID NO: 3:
   (i) SEQUENZ CHARAKTERISTIKA:
      (A) LÄNGE: 29 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: JA
   (iii) ANTISENSE: NEIN
   (xi) SEQUENZBESCHREIBUNG: SEQ ID NO: 3:
   TTGGAATTCA TCCCNNNNNN CCACACATC 29
(2) INFORMATION ZU SEQ ID NO: 4:
   (i) SEQUENZ CHARAKTERISTIKA:
      (A) LÄNGE: 24 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: JA
   (iii) ANTISENSE: NEIN
   (xi) SEQUENZBESCHREIBUNG: SEQ ID NO: 4:
   TTTGGAATTC GTNCAYMGNG AYYT 24
(2) INFORMATION ZU SEQ ID NO: 5:
   (i) SEQUENZ CHARAKTERISTIKA:
      (A) LÄNGE: 37 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: JA
   (iii) ANTISENSE: NEIN
   (xi) SEQUENZBESCHREIBUNG: SEQ ID NO: 5:
   GCAGAATTCG TGAACTGCGG CCGCATTTTT TTTTTTT 37
(2) INFORMATION ZU SEQ ID NO: 6:
   (i) SEQUENZ CHARAKTERISTIKA:
      (A) LÄNGE: 22 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: JA
   (iii) ANTISENSE: NEIN
   (xi) SEQUENZBESCHREIBUNG: SEQ ID NO: 6:
   CACCTCGAAA CTGTGCTCTT TC 22
(2) INFORMATION ZU SEQ ID NO: 7:
   (i) SEQUENZ CHARAKTERISTIKA:
      (A) LÄNGE: 2503 Basenpaare
      (B) ART: Nukleinsäure
      (C) STRANGFORM: beides
      (D) TOPOLOGIE: linear
   (ii) ART DES MOLEKÜLS: cDNS
   (iii) HYPOTHETISCH: NEIN
   (iii) ANTISENSE: NEIN
   (vi) URSPRÜNLICHE HERKUNFT:
      (A) ORGANISMUS: Homo sapiens
   (xi) SEQUENZBESCHREIBUNG: SEQ ID NO: 7:
   CTAACGGGTG TGGTGGTGTG CACCTGTAGT CCCAGCTACT CGGGAGGCTG AGGCAGGAGA 60
   ATCGCTTGAA CCCAGGAGGT GGAGGCTGCA GTGAGCCAAG ATCACGCCAC TGCACTCCAG 120
   CCTGGGTGAC AGAGCAAGAC TCCATCTCAA CAACAACAAC AAAAAGGAAT TAAGGAGGAA 180
   GCCACCTGGC TTCCTAAGAT AGACTTTATG GTTAATGGGA TTTATTTTAG CTGGTTAAGT 240
   CCAGGACCCC TACCAGGAAG AACTGAGGTC CATATTTGAG GTTGGTCTCA CCCTTTTGCA 300
   TGTGGTCCTA CTAATAATAA TTTTGCTGTA AAGAAGCCTC AATAAGGAGA TGGACTGTGT 360
   GAAAAGAGAT GAGAAAGCAG GCCTGTTTGC AAGGCTGGCC CTTGGCTGGC ATCTGAAAGC 420
   TTGGATTTCA GGAGGGTTCT CATTACTCCC TAAATGATAA GAGTGGCTCA CTGTGCCTAA 480
   ACTGTTCGTA CAAACAATGT GGGTTATGCC CAACATCTGC TTTCCTTCTG GGAGTCAGAT 540
   TTTCATATGT GTTAGGCAGA GGATGAGGTG AGCAGCTCCC AATGAGAACC CTGAACACTG 600
   AGTCTGTAAT GAGCTTCCCT TGTATACAAC ATTGCACATG GGTTGTCACA ACTGATTGCT 660
   GGAGGAATTG TGTCCTATGT GACTCTGCTG GGAGAGGACT GTGGGAGGCT TACACCTGGT 720
   TTCCCTGGAC TTTGTCCATG CGCTTTTTTC CTTTGCTGAT TTTGCTTCCT AGCCTTTCGC 780
   TGTAGTAAAA CATAGCCATG AGTATGACTA CAGGCTGAGT CTGTGAATCT CCTAGTACAT 840
   CATCAGACTA GGAGGTGGTG GTGTTGGCAC CCCCCAGCAC AGGGCACAAG GGAGACTTGC 900
   AGGGTGTTTG TCATGTTCCC TTTCTCAATC TGGGTGTGGT TTACAGATTT GTTCAGTTTG 960
   TGAAAATTCA CTGAGCTCTA TGAACAATTA TAATATGTAC ATTTTTTTCT GTAAGTGTAT 1020
   TAAATTTCAA TAAAAAGAAT TCACACTATA GGGGTACAAA ATAACTTAAT TTTTCAAAGT 1080
   CTTCCTTTTA TTCAGCCAAC ATTTTAGTAA GTACCCTTTT TTTTTTACCA AGCACTGCTC 1140
   TGGGAGCTTG GGACATGGGG TAGAAAGGAC GGCATCATTG TCCTCATTGT CCTTCAGCTT 1200
   AGCTGCAAGG CAGGTGAAGG ATGCTTATAG TATGGTTGAT TTTTGCTGGG CTCTATGAAG   1260
   GATCAGAGGG TGCTAAGGAG GCACAGAGAA AGAGCTTTTA ACGTGGGTAT TTTACTGTTC   1320
   CAGGGTTGCC ACTCAAGTAC CTACAGAACA AACGGGTGTA GCTGTAAACT GTGGCAAACT   1380
   GAAAGCATGT AGGCACAGAG TAAGTAGCTA GAGCTGATTT TTTTCAGGAA AGGCCACAAA   1440
   TCCCAACTTT TATGAGCTCC TCTTTTAAAT GCTGTAAATG TTTTACAATG GCGGGGCGCG   1500
   GTGGCTCACG CCTGTAATCC CAGCATTTGG GAGGCCGAGG GGCAGATCAC TTGAGGTCAG   1560
   GAGTTCAAGA CCAGCCTGGC CAACATGGTG AAACCTCTTC TCTACTAAAA TTACAAAAAT   1620
   TAGCCGGGCA TGGTGGTGCA TGCTTGTAAT TCCAGCTGCT AGGGAGGCCG AGGCGGGAGG   1680
   ATTGCTTGAA CCCGGGAAGC AGAGGTTGCA GTGAGCTGAG ATCGTGCCAC TGCACTCCAG   1740
   CCTGGGCGAC AGAGCAAGAT TCCGTCACAC ACACAAAAAA AAGGCGTGGG GGGAGGCCAA   1800
   ACAAAACCCC GCAAGACACA TTTGGCTATG ACCTGCCAGT TTGCTAGGCA TTCTTCCAAC   1860
   CTTCCCTCCC TCTGACCAAG AAACTGAGTG TCCACTATTT TAGGCCCTGG GAAATTCAGT   1920
   AGCGAGGAGG CCAGACAGCT TCGTTGCATC ATGGGGGGCT CTGGTACTGT GCTTCTCCAA   1980
   CTTCAGGATG TGTAGGAATC ACCTGAGCAG TCTTGTTGAG AGGCGGACAC TGACTCGGGA   2040
   GGTCTGGGGT AGGGCCTGAA CGTTTGCCTT TGCGGTTCTA ACAAGCTCTC AGGTGATGGC   2100
   GATGCTACTG TTCCCTGGCC CCGAGGTAGA GGAAGATTTA AGTAAAAGCT TCCTGGAGGA   2160
   GGCGCAAGTG AACCGCAGGA GCTTTCCCGG ACGCCCGAGA AGGGAGAAAC CCCGAAGGAA   2220
   TTCCTCCTCT CTCGGGGCTG GGTCTCCGCA TCCACGCCGG GTTTGGTTTC CCAGGCTATC   2280
   CCACGTGTTC GGGCGTCCGT GTCAATCAGG TTTTCCCCGG CTGGGTCCGG GTTTAAAGGC   2340
   TGCTGCTGCG CAGGGCGCTC CCATGGTGCC GCGCGGCGGG CGGGTTTGGA TTTTAAATCC   2400
   CCGCGGCCAA TCAGTGGCGC GCAGGCTTTT GTAACGTTCC CAGCGCCGCG TTTGAATTCG   2460
   GGGAGGAGCG GAGCGGTGCG GAGGCTCTGC TCGGATCGAG GTC                    2503